# EUROPEAN PATENT SPECIFICATION

(11) **EP 1 917 954 B1**
(45) Date of publication and mention of the grant of the patent: **21.02.2018**
(21) Application number: 06021516.7
(22) Date of filing: 13.10.2006
(51) Int. Cl.: A61K 8/06

(54) **Aqueous meta-stable oil-in-water emulsions**
Wässrige metastabile Öl-in-Wasser Emulsionen
Emulsions métastables de type huile-dans-eau

(43) Date of publication of application: 07.05.2008
(73) Proprietor: Cognis IP Management GmbH, 40589 Dusseldorf (DE)
(72) Inventor: Amela Conesa, Cristina, 08290 Cerdanyola del Vallés (ES); Domingo, Marta, 08024 Barcelona (ES); Beuché, Marc, 91430 Vauhallan (FR)
(74) Representative: BASF IP Association

(56) References cited:
- WO-A-2004/050044
- DE-A1- 10 252 757
- US-A- 4 551 330
- US-A- 4 781 914
- US-A1- 2004 219 123

## Description

### Object of the invention

The present invention relates to the area of cosmetic and pharmaceutical compositions and more particularly concerns a process for the preparation of low viscous, meta-stable o/w emulsions which invert into w/o emulsions when applied to skin.

### State of the art

As water in oil (w/o) emulsion systems are close to the skin's hydrolipid film, they are more effective from the dermatological viewpoint. This emulsion type promotes the long lasting moisturizing efficacy by providing an occlusive film and reinforces the active ingredients action into the stratum corneum. In addition, w/o emulsions leave a lipophilic film on the skin surface which ensures high water repellency, which is an important parameter to maintain high UV protection in sun care applications. Nevertheless, tackiness, combined with greasiness and slow spreading are key factors which tend to decrease cosmetic acceptance, thus counteracting w/o emulsion benefits.

European patent application EP 1174180 B1 (Clariant) claims a process for preparing fine emulsions showing a particle size in the range from 0.1 to 10 µm which are characterised in that a w/o pre-emulsion, comprising at least one w/o emulsifier selected from sorbitol esters, polyglycerol esters, sorbitan esters, fatty acid esters and/or dimethiconcopolyols are treated with at least one surfactant in order to invert said w/o fine emulsion into an o/w fine emulsion on condition that the inversion is conducted without increasing the temperature. However, the emulsions thus obtained do not exhibit a sufficient stability, especially when stored at higher temperatures over a couple of days. In addition, the process requires the preparation of an intermediate w/o emulsion which is turned into an o/w emulsion which makes the manufacture time-consuming and little efficient.

Reference is also made to DE 10252757 A1 (Beiersdorf) disclosing care formulations, more particularly O/W emulsions which switch to W/O emulsions when applied to the skin. These products, however, are free of anionic surfactants. US 4,781,914 (Charles of the Ritz) discloses a process for switch emulsions, however, the W/O emulsifier is incorporated into the aqueous phase. Finally, US 4,551,330 (Helene Curties) refers to inverting emulsions comprising various metal ions. None of the processes cited in these documents disclose a manufacturing process which allows obtaining switch emulsions of satisfying properties.

Therefore, the complex problem underlying the present invention has been to develop a new process to obtain o/w emulsions exhibiting
- viscosities low enough to be formulated as sprays;
- long lasting moisturising effect;
- high water resistance;
- enhanced UV protection;
- improved active ingredients efficacy;
- stability under normal storage conditions;
- but inverting into a w/o emulsion when applied to skin
while at the same time avoiding the disadvantages known from the state of the art.

### Description of the invention

The present invention refers to a first process for the preparation of aqueous meta-stabile o/w emulsions with the ability to invert into w/o emulsions by introduction of mechanical energy obtainable by the following method:
(i) oil components, w/o emulsifiers and optionally oil-soluble actives are mixed at temperatures in the range from 50 to 90° C to form an oil phase I,
(ii) anionic surfactants, selected from the group consisting of acyl glutamates, alk(en)yl oligoglycoside carboxylates, alk(en)yl polyalkyleneglycolether citrates and their mixtures, are diluted in water at temperatures in the range from 50 to 90° to form an aqueous phase, and this phase is maintained at these temperatures (II)
(iii) phase (I) is added to phase (II) or, alternatively, phase II is added to phase I while maintaining these temperatures in order to obtain an o/w emulsion,
(iv) said o/w emulsion is cooled down to room temperature,
(v) polymeric stabilisers and optionally water soluble actives are added, and
(vi) the final emulsion is homogenised.

In a preferred embodiment of the present invention, the polymeric stabiliser is present during the emulsification process.

The present invention also concerns a second process for the preparation of aqueous meta-stabile o/w emulsions with the ability to invert into w/o emulsions by introduction of mechanical energy obtainable by the following method:
Oil components, w/o emulsifiers and optionally oil-soluble actives and/or polymeric stabilisers are mixed at temperatures in the range from 50 to 90° to form an oil phase I, anionic surfactants selected from the group consisting of acyl glutamates, alk(en)yl oligoglycoside carboxylates, alk(en)yl polyalkyleneglycolether citrates and their mixtures and optionally polymeric stabilisers are diluted in water at temperatures in the range from 50 to 90° to form an aqueous phase (II), phase (I) is added to phase (II) while maintaining these temperatures in order to obtain an o/w emulsion, said o/w emulsion is cooled down to room temperature, optionally water soluble actives are added, and the final emulsion is homogenised on condition that said polymeric stabilisers are present in the oil or aqueous phase or both.

The temperatures are preferably from about 60 to about 80 °C.

Surprisingly it has been found that the compositions produced according to the present invention allow the formulation of stable and sprayable sun care o/w emulsions, the interest of which lies in their capacity to invert very quickly into a w/o emulsion while rubbing on the skin. The formation of a lipophylic film on the skin surface is much faster than the normal inversion process that takes place when a standard o/w emulsion is applied and the water phase evaporates. The sprayable emulsion permits to claim a water resistance effect without including any waterproof ingredients. This allows boosting the long lasting SPF protection without wash-off while bathing. The experiments supporting the present invention also conclude the ability of the compositions to reduce TEWL showing long-term moisturising effect of epidermis. Therefore, this specific emulsifying system is applicable in all those applications where a high skin protection is required (for example body lotion for dry skin, care lotion for babies and children). Finally, and endorsed by its positive sensory profile (mainly in terms of absorption, tackiness and smoothness), this in-situ w/o emulsion generates the sensory characteristics of an o/w emulsion with the benefits of a w/o one.

### Oil components

Suitable oil components (component a) are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈- alkylhydroxy carboxylic acids with linear or branched C₆-C ₂₂-fatty alcohols, in particular Dioctyl Malate, esters of linear and/or branched fatty acids with polyhydric alcohols (such as, for example, propylene glycol, dimerdiol or trimertriol) and/or Guerbet alcohols, triglycerides based on C₆ -C₁₀-fatty acids, liquid mono-/di-/triglyceride mixtures based on C₆-C₁₈-fatty acids, esters of C₆- C₂₂-fatty alcohols and/or Guerbet alcohols with aromatic carboxylic acids, in particular benzoic acid, esters of C₂- C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol® CC), Guerbet carbonates, based on fatty alcohols having 6 to 18, preferably 8 to 10, carbon atoms, esters of benzoic acid with linear and/or branched C₆-C₂₂-alcohols (e.g. Finsolv® TN), linear or branched, symmetrical or asymmetrical dialkyl ethers having 6 to 22 carbon atoms per alkyl group, such as, for example, dicaprylyl ether (Cetiol® OE), ring-opening products of epoxidized fatty acid esters with polyols, silicone oils (cyclomethicones, silicone methicone grades, etc.), aliphatic or naphthenic hydrocarbons, such as, for example, squalane, squalene or dialkylcyclohexanes, and/or mineral oils. The preferred oil components, which give the highest benefit from an applicational point of view, can be chosen from the group consisting of tri- or partial glycerides, dialkyl ethers, dialkyl carbonates, liposoluble UV filters like, for example, chemicals filters and/or solutions of pigments, preferably TiO₂ in cosmetic oils, and their mixtures.

### W/O Emulsifiers

Typically, the w/o emulsifiers (component b) are chosen from the group consisting of sorbitan esters and polyglycerol esters:

### • Sorbitan esters

Suitable sorbitan esters are sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 mol, and preferably 5 to 10 mol, ethylene oxide onto the sorbitan esters mentioned are also suitable.

### • Polyglycerol esters

Typical examples of suitable polyglycerol esters are Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) and Polyglyceryl Polyricinoleate (Admul® WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

### Anionic surfactants

The nature of the anionic surfactants (component c) is more critical as by far not all types show similar effects with respect to stability and the power to induce a spontaneous phase inversion of the emulsion when applied to skin. Anionic surfactants are selected from the group consisting of N-acyl amino acids, in particular acyl glutamates, alk(en)yl oligoglycoside carboxylates, polyglycolether citrates and their mixtures.

### • N-acylamino acids

Basically, the N-acylamino acids which form component (c1) may be derived from any α-amino acids which can be acylated with fatty acid halides to form N-acylamino acids. Preferred amino acids are glutamic acid, sarcosine, aspartic acid, alanine, valine, leucine, isoleucine, proline, hydroxyproline, lysine, glycine, serine, cystein, cystine, threonine, histidine and salts thereof and, more particularly, glutamic acid, sarcosine, aspartic acid, glycine, lysine and salts thereof. The amino acids may be used in optically pure form or as racemic mixtures. The amino acid components of the N-acylamino acids are preferably derived from glutamic acid and/or aspartic acid, i.e. N-acyl glutamates and N-acyl aspartates are preferably used. In addition, the acyl groups of the N-acylamino acids may be derived from fatty acids corresponding to formula **(I):**

**R¹CO-OH** **(I)**

in which R¹ is a linear or branched acyl group containing 6 to 22 carbon atoms and 0 and/or 1 to 3 double bonds. Typical examples are acyl groups derived from caproic acid, caprylic acid, capric acid, lauric acid, mrystic acid, palmitic aid, stearic acid, isostearic acid, oleic acid, elaidic acid, linoleic acid, linolenic acid, gadoleic acid, arachidonic acid, behenic acid and erucic acid and technical mixtures thereof. The N-acylamino acids are preferably derived from technical C₁₂₋₁₈ coconut oil fatty acids. The N-acylamino acids may be present in acidic form, but are generally used in the form of their salts, preferably alkali metal or ammonium salts. The sodium and triethanolamine salts are particularly preferred. Overall, N-cocoyl glutamate is the preferred N-acylamino acid.

### Alk(en)yl oligoglycoside carboxylates

The alk(en)yl oligoglycoside carboxylates which can be used in the compositions produced according to the invention as component (c2) correspond to formula **(II) :**

**R²O[G]ₚO[(CH₂)ₘCOO⁻X⁺]ₙ** **(II)**

in which R² is an alkyl or alkenyl radical having from 6 to 22 carbon atoms, G is a sugar unit having 5 or 6 carbon atoms, p is a number from 1 to 10, m and n are numbers from 1 to 5, and X is alkali, alkaline earth, ammonium, alkylammonium, alkanolammonium or glucammonium. The products are obtainable according to the methods known from the art, for example by reaction of alk(en)yl oligoglycosides with halogen carboxylic acids or their salts in alkaline medium in the presence of solvents. The alkyl or alkenyl oligoglycosides carboxylates may be derived from aldoses or ketoses containing 5 or 6 carbon atoms, preferably glucose. Accordingly, the preferred alkyl and/or alkenyl oligoglycoside carboxylates are alkyl or alkenyl oligo**glucoside** carboxylates. The index p in general formula **(II)** indicates the degree of oligomerisation (DP degree), i.e. the distribution of mono- and oligoglycosides, and is a number from 1 to 10. Whereas p in a given compound must always be an integer and, above all, may assume a value of 1 to 6, the value p for a certain alkyl oligoglycoside moiety is an analytically determined calculated quantity which is mostly a broken number. Alk(en)yl oligoglycoside carboxylates having an average degree of oligomerisation p of 1.1 to 3.0 are preferably used. Alk(en)yl oligoglycoside carboxylates having a degree of oligomerisation below 1.7 and, more particularly, between 1.2 and 1.4 are preferred from an applicational point of view.

The alkyl or alkenyl radical R² may be derived from primary alcohols containing 4 to 22 carbon atoms, and preferably 8 to 16 carbon atoms. Typical examples are butanol, caproic alcohol, caprylic alcohol, capric alcohol, undecyl alcohol, lauryl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and technical mixtures thereof such as are formed, for example, in the hydrogenation of technical fatty acid methyl esters or in the hydrogenation of aldehydes from Roelen's oxo synthesis. Alkyl oligoglucosides based on hydrogenated C₈-C₁₆ coconut oil alcohol having a DP of 1 to 3 are preferred.

Further on, the alk(en)yl oligoglycoside carboxylates may be derived from carboxylic acids, their salts or esters, in which the acyl moiety comprises 1 to 5, preferably 2 to 4 and more preferably 1 or 2 carbon atoms, while the number of acyl groups in the alk(en)yl oligoglycoside carboxylate may be 1 to 5 and preferably 1 to 3. Moreover, X stands preferably for potassium, ammonium, triethanolammonium and most preferably for sodium. The carboxylic acids, which can be used for preparing the alk(en)yl oligoglycoside carboxylates, usually comprise 1 to 4 carbon atoms; preferably acetic acid, its esters or its salts, particularly its sodium or potassium salt are used. In a preferred embodiment of the present invention, alk(en)yl oligoglycoside carboxylates are used which are obtained by reaction of an aqueous solution of an alk(en)yl oligoglycoside, having e.g. 20 to 70 % b.w. solids matter, under nitrogen and in presence of an alkaline catalyst, e.g. alkali hydroxide or alkali carbonate, at a temperature of from 50 to 100 °C with ω-halogen carboxylic acid, its ester or salt, like for example potassium or sodium monocloroacetate in a molar ratio of from 1:0.5 to 1:5, preferably 1:1 to 1:3. The molar ratio of alkali:halogen carboxylic acid, its esters or salts is usually adjusted to 1:0.5 to 1:1.5 and preferably 1:1.1. The preparation of the medium chain C_{12/14} alkyl oligoglycoside carboxylates usually takes place in the absence of organic solvents, while the long chain C_{16/18} alkyl oligoglycoside carboxylates are prepared in the presence of long chain fatty alcohols or 1.2-propylene glycol. These products are obtainable in the market, for example, under the trademark Plantapon® LGC (Cognis Deutschland GmbH & Co. KG).

### • Alkylpolyglycolether citrates

Alk(en)yl polyalkylene glycol ether citrates, which form component (c3), represent mixtures of mono, di and triesters of citric acid and alkoxylated alcohols following formula **(III)** 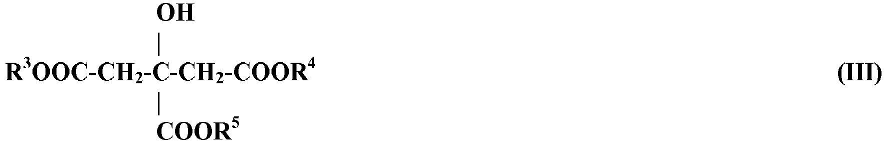 in which R³, R⁴, and R⁵ independently stand for hydrogen or a radical **(IV)**

**R⁶(OCH₂CHR⁷)ₙ** **(IV)**

in which R⁶ is a linear or branched alkyl and/or alkenyl group containing 6 to 22 carbon atoms, R⁷ is hydrogen or methyl, and n is a number from 1 to 20, under the condition that at least R³, R⁴, or R⁵ is different from hydrogen. Typical examples for the alcohol part of the esters are the addition products of on average 1 to 20 moles, and more particularly, 5 to 10 moles of ethylene oxide and/or propylene oxide onto caproic alcohol, caprylic alcohol, 2-ethyl hexyl alcohol, capric alcohol, lauryl alcohol, isotridecyl alcohol, myristyl alcohol, cetyl alcohol, palmitoleyl alcohol, stearyl alcohol, isostearyl alcohol, oleyl alcohol, elaidyl alcohol, petroselinyl alcohol, arachyl alcohol, gadoleyl alcohol, behenyl alcohol, erucyl alcohol and brassidyl alcohol and technical mixtures thereof. The ethers may have both a conventional homologue distribution and a narrow homologue distribution. It is particularly preferred to use alkyl polyalkylene glycol ethers based on addition products of, on average, 5 to 10, and more particularly of about 7 moles of ethylene oxide with technical C₁₂-C₁₈, preferably C₁₂-C₁₄ fatty alcohol fractions. These products are obtainable in the market, for example, under the trademark Plantapon® LC7 (Cognis Deutschland GmbH & Co. KG).

### Actives

The nature of the active ingredients (component d) is not critical since the selection fully depends on the required properties of the final product. Typically, the actives are selected from the group consisting of antioxidants, pigments, plant extracts and UV filters (primary and secondary sun protection factors), which are explained in more detail in the following:

### • Primary sun protection factors

Primary sun protection factors in the context of the invention are, for example, organic substances (light filters) which are liquid or crystalline at room temperature, and which are capable of absorbing ultraviolet radiation and of releasing the energy absorbed in the form of longer-wave radiation, for example heat. UV-B filters can be oil-soluble or water-soluble. The following are examples of oil-soluble substances:
∘ 3-benzylidene camphor or 3-benzylidene norcamphor and derivatives thereof, for example 3-(4-methylbenzylidene)-camphor;
∘ 4-aminobenzoic acid derivatives, preferably 4-(dimethylamino)benzoic acid-2-ethylhexyl ester, 4-(dimethylamino)-benzoic acid-2-octyl ester and 4-(dimethyl-amino)benzoic acid amyl ester;
∘ esters of cinnamic acid, preferably 4-methoxycinnamic acid-2-ethylhexyl ester, 4-methoxycinnamic acid propyl ester, 4-methoxycinnamic acid isoamyl ester, 2-cyano-3,3-phenylcinnamic acid-2-ethylhexyl ester (Octocrylene);
∘ esters of salicylic acid, preferably salicylic acid-2-ethylhexyl ester, salicylic acid-4-isopropylbenzyl ester, salicylic acid homomenthyl ester;
∘ derivatives of benzophenone, preferably 2-hydroxy-4-methoxybenzophenone, 2-hydroxy-4-methoxy-4'-methylbenzophenone, 2,2'-dihydroxy-4-methoxybenzophenone;
∘ esters of benzalmalonic acid, preferably 4-methoxybenzalmalonic acid di-2-ethylhexyl ester;
∘ triazine derivatives such as, for example, 2,4,6-trianilino-(p-carbo-2'-ethyl-1'-hexyloxy)-1,3,5-triazine and Octyl Triazone or Dioctyl Butamido Triazone (Uvasorb® HEB);
∘ propane-1,3-diones such as, for example, 1-(4-tert.butylphenyl)-3-(4'-methoxyphenyl)-propane-1,3-dione;
∘ ketotricyclo(5.2.1.0)decane derivatives.

### Suitable water-soluble substances are

∘ 2-phenylbenzimidazole-5-sulfonic acid and alkali metal, alkaline earth metal, ammonium, alkylammonium, alkanolammonium and glucammonium salts thereof;
∘ sulfonic acid derivatives of benzophenones, preferably 2-hydroxy-4-methoxybenzophenone-5-sulfonic acid and salts thereof;
∘ sulfonic acid derivatives of 3-benzylidene camphor such as, for example, 4-(2-oxo-3-bornylidenemethyl)-benzene sulfonic acid and 2-methyl-5-(2-oxo-3-bornylidene)-sulfonic acid and salts thereof.

Typical UV-A filters are, in particular, derivatives of benzoyl methane such as, for example, 1-(4'-tert.butylphenyl)-3-(4'-methoxyphenyl)-propane-1,3-dione, 4-tert.butyl-4'-meth-oxydibenzoyl methane (Parsol® 1789) or 1-phenyl-3-(4'-isopropylphenyl)-propane-1,3-dione and the enamine compounds (BASF). The UV-A and UV-B filters may of course also be used in the form of mixtures. Particularly favourable combinations consist of the derivatives of benzoyl methane, for example 4-tert.butyl-4'-methoxydibenzoyl methane (Parsol® 1789) and 2-cyano-3,3-phenylcinnamic acid-2-ethylhexyl ester (Octocrylene®), in combination with esters of cinnamic acid, preferably 4-methoxycinnamic acid-2-ethylhexyl ester and/or 4-methoxycinnamic acid propyl ester and/or 4-methoxycinnamic acid isoamyl ester. Combinations such as these are advantageously combined with water-soluble filters such as, for example, 2-phenylbenzimidazole-5-sulfonic acid and alkali metal, alkaline earth metal, ammonium, alkylammonium, alkanolammonium and glucammonium salts thereof.

### • Secondary sun protection factors

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the skin. Typical examples are amino acids (for example glycine, histidine, tyrosine, tryptophane) and derivatives thereof, imidazoles (for example urocanic acid) and derivatives thereof, peptides such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in oils, water or ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

### • Biogenic agents

In the context of the invention, biogenic agents are, for example, tocopherol, tocopherol acetate, tocopherol palmitate, ascorbic acid, (deoxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example prune extract, bambara nut extract, and vitamin complexes.

### Polymeric stabilisers

Suitable polymeric stabilisers (component e) can be selected from the group consisting of polyacrylates and polysaccharides. Typical examples for products found in the market are Cosmedia® SP, Cosmedia® SPL, Rheocare® ATH or Keltrol® T.

### Meta-stable o/w emulsions

In a preferred embodiment of the present invention, the meta-stable o/w emulsions show the following composition:

| | | | | | | | | |
|---|---|---|---|---|---|---|---|---|
| (a) | 10 | to | 35, | preferably | 10 | to | 30 | % b.w. oil components, |
| (b) | 1 | to | 7, | preferably | 2 | to | 5 | % b.w. w/o emulsifiers, |
| (c) | 0.1 | to | 5, | preferably | 0.5 | to | 3.5 | % b.w. anionic surfactants, |
| (d) | 0.1 | to | 10, | preferably | 0.5 | to | 8 | % b.w. actives, |
| (e) | 0.05 | to | 1.5, | preferably | 0.5 | to | 1 | % b.w. polymeric stabilisers |

on condition that the components add up to 100 % b.w with water and optionally other typical cosmetic ingredients. The emulsions according to the present invention show a viscosity according to Brookfield of less than 3,000 mPas (20 °C, spindle 5, 10 rpm) which allows them to be applied as a spray. Nevertheless, by adding thickeners it is possible to obtain also creams or lotions showing similar switching behaviour.

### Industrial application

A final embodiment of the present invention refers to the use of said meta-stable o/w emulsions for making cosmetic and/or pharmaceutical compositions, in particular for making skin care compositions, or hair care compositions, or (water-resistant) sun care compositions, which may be present in the form of a lotion or a spray. For each specific purpose, the final products may comprise additional ingredients, like for example additional surfactants, additional oil bodies, additional emulsifiers, superfatting agents, pearlizing waxes, consistency factors, polymers, silicone compounds, waxes, stabilizers, hydrotropes, preservatives, solubilizers, perfume oils, dyes and the like.

### Superfatting agents

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

### Consistency factors

The consistency factors mainly used are fatty alcohols or hydroxyfatty alcohols containing 12 to 22 carbon atoms, preferably 16 to 18 carbon atoms, and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### Thickening agents

Suitable thickeners are polymeric thickeners, such as Aerosil® types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols® [Goodrich] or Synthalens® [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### Polymers

Suitable cationic polymers are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quaternized vinyl pyrrolidone/vinyl imidazole polymers such as, for example, Luviquat® (BASF), condensation products of polyglycols and amines, quaternized collagen polypeptides such as, for example, Lauryldimonium Hydroxypropyl Hydrolyzed Collagen (Lamequat® L, Grünau), quaternized wheat polypeptides, polyethyleneimine, cationic silicone polymers such as, for example, amodimethicone, copolymers of adipic acid and dimethylaminohy-droxypropyl diethylenetriamine (Cartaretine®, Sandoz), copolymers of acrylic acid with dimethyl diallyl ammonium chloride (Merquat® 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar® CBS, Jaguar® C-17, Jaguar® C-16 of Celanese, quaternized ammonium salt polymers such as, for example, Mirapol® A-15, Mirapol® AD-1, Mirapol® AZ-1 of Miranol.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### Pearlizing waxes

Suitable pearlizing waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxy-substituted carboxylic acids with fatty alcohols containing 6 to 22 carbon atoms, especially long-chain esters of tartaric acid; fatty compounds, such as, for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### Silicones

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976**).**

### Waxes

Besides natural oils used, waxes may also be present in the preparations, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

### Stabilizers

Metal salts of fatty acids such as, for example, magnesium, aluminium and/or zinc stearate or ricinoleate may be used as stabilizers.

### Hydrotropes

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### Preservatives

Suitable preservatives are, for example, phenoxyethanol, formaldehyde solution, parabens, pentanediol or sorbic acid and the other classes of compounds listed in Appendix 6, Parts A and B of the Kosmetikverordnung ("Cosmetics Directive").

### Complexing agents

The complexing agents used may be selected from EDTA, NTA, phosphonic acids, Triton B, turpinal and phenacetin. In addition, reducing agents such as, for example, ascorbic acid, sodium sulfate, sodium thiosulfate and the like may be present. Suitable alkalizing agents are ammonia, monoethanolamines, (L) arginine, AMP, etc.

### Perfume oils

Suitable perfume oils are mixtures of natural and synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, □-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### Dyes

Suitable dyes are any of the substances suitable and approved for cosmetic purposes as listed, for example, in the publication **"**Kosmetische Färbemittel" of the Farbstoffkommission der Deutschen Forschungsgemeinschaft, Verlag Chemie, Weinheim, 1984, pages 81 to 106**.** Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. These dyes are normally used in concentrations of from 0.001 to 0.1% by weight, based on the mixture as a whole.

The total percentage content of auxiliaries and additives may be from 1 to 50% by weight and is preferably from 5 to 40% by weight, based on the particular composition.

### Examples

### Examples 1 to 10

### Meta-stable o/w emulsions (amounts calculated as % b.w.)

| **Phase** | **Compound** | **1** | **2** | **3** | **4** | **5** | **6** | **7** | **8** | **9** | **10** |
|---|---|---|---|---|---|---|---|---|---|---|---|
| **A** | **Dehymuls**® **PGPH** | 4.0 | 4.0 | 4.0 | 4.0 | 4.0 | 4.0 | 4.0 | 4.0 | 4.0 | 4.0 |
| | Polyglyceryl-2 Dipolyhydroxystearate | | | | | | | | | | |
| | **Cetiol**® **CC** | 2.0 | 2.0 | 4.0 | 4.0 | 4.0 | 4.0 | 4.0 | 4.0 | 4.0 | 9.0 |
| | Dicaprylyl Carbonate | | | | | | | | | | |
| | **Cetiol**® **B** | 2.0 | 2.0 | | | | | | | | |
| | Dibutyl Adipate | | | | | | | | | | |
| | **Myritol**® **331** | 2.0 | 2.0 | | | | | | | | 6.0 |
| | Cocoglycerides | | | | | | | | | | |
| | **Cegesoft**® **PS 6** | | | | | | | | | | 3.0 |
| | Vegetable Oil | | | | | | | | | | |
| | **DC 245** | | | 4.0 | 4.0 | 4.0 | 4.0 | 2.0 | 4.0 | 2.0 | |
| | Cyclomethicone | | | | | | | | | | |
| | **DC 200** | | | | | | | | | | 0.5 |
| | Hexamethyldisiloxane | | | | | | | | | | |
| | **Neo Heliopan® AV** | 7.0 | 7.0 | 7.0 | 7.0 | 7.0 | 7.0 | 7.0 | 7.0 | 7.0 | |
| | Octyl Methoxycinnamate | | | | | | | | | | |
| | **Neo Heliopan® E 1000** | 6.0 | 6.0 | 6.0 | 6.0 | 6.0 | 6.0 | 6.0 | 6.0 | 6.0 | |
| | Isoamyl p-Methoxycinnamate | | | | | | | | | | |
| | **Neo Heliopan® 357** | 1.5 | 1.5 | 1.5 | 1.5 | 1.5 | 1.5 | 1.5 | 1.5 | 1.5 | |
| | Methyl Butoxydibenzoylmethane | | | | | | | | | | |
| | **Neo Heliopan® MBC** | 3.5 | 3,5 | 3,5 | 3.5 | 3.5 | 3,5 | 3,5 | 3.5 | 3.5 | |
| | 4-Methyl Benzylidene Camphor | | | | | | | | | | |
| | **Cosmedia® DC** | 2.0 | 2.0 | | | | | | | | |
| | Hydrogenated Dimer Dilinoleyl/ Dimethylcarbonate Copolymer | | | | | | | | | | |
| | **Cosmedia® SP** Sodium Polyacrylate | | | | | 0.6 | | | | | |
| **B** | **Water** | | | | | ad to | 100 | | | | |
| | **Plantapon® ACG 35** | 1.5 | 1.5 | 3.0 | 3.0 | | | | | 1.1. | |
| | Sodium Cocoyl Glutamate | | | | | | | | | | |
| | **Plantapon® LGC sorb** | | | | | 1.5 | 1.5 | 1.5 | | | 1.5 |
| | Sodium Lauryl Glucose Carboxylate (and) Lauryl Glucoside | | | | | | | | | | |
| | **Plantapon® LC7** Laureth-7 Citrate | | | | | | | | 3.0 | | |
| | **NaOH (10 % b.w.)** | | | | | | | | 0.6 | | |
| | **Glycerol** | 3.0 | 3.0 | 3.0 | 3.0 | 3.0 | 3.0 | 3.0 | 3.0 | 3.0 | 3.0 |
| | **Phenonip** | 0.3 | 0.3 | 0.3 | 0.3 | 0.3 | 0.3 | 0.3 | 0.3 | 0.3 | 0.3 |
| | **Cosmedia® SP** | | | | | | 0.6 | 0.6 | | | 0.6 |
| | Sodium Polyacrylate | | | | | | | | | | |
| **C** | **Cosmedia SPL** | | | | | | | | 1.5 | 0.8 | |
| | Sodium Polyacrylate (and) Hydrogenated Polydecene (and) PPG-5 Laureth-5 | | | | | | | | | | |
| **D** | **Tioveil® AQ-N** | | | 14.0 | 14.0 | | | | | | |
| | Titanium Dioxide (and) Aluminia (and) Silica (and) Sodium Polyacrylate | | | | | | | | | | |
| | **Cetiol® Sun** | | | | | | | 14.0 | | | |
| | Titanium Dioxide (and) Dioctyl Carbonate | | | | | | | | | | |
| | **Ethanol** | 5.0 | 5.0 | 5.0 | 5.0 | 5.0 | 5.0 | 5.0 | 5.0 | 5.0 | 5.0 |
| | **Keltrol® T** | 0.4 | 0.2 | 0.3 | 0.2 | | | | | | |
| | Xanthan Gum | | | | | | | | | | |
| | **Tocopheryl Acetate** | 0.5 | 0.5 | 0.5 | 0.5 | 0.5 | 0.5 | 0.5 | 0.5 | 0.5 | 0.5 |
| | **Perfume** | 0.2 | 0.2 | 0.2 | 0.2 | 0.2 | 0.2 | 0.2 | 0.2 | 0.2 | 0.2 |

The compositions according to Examples 1, 2, 3, 4, 5, 6, 7 and 10 were prepared by heating the ingredients of phase A to about 80 °C in order to obtain an oil phase I. The ingredients of phase B were also mixed and heated to about 80 °C in order to obtain an aqueous phase II. Subsequently phase I was given to phase II without cooling in order to obtain an o/w phase. Once the resulting product was cooled down to about 25 °C, the ingredients of phase D were added. Finally, the emulsion was homogenised. The compositions according to Examples 8 and 9 were prepared in the same way, with the exception that phase C was added along with phase D to the o/w emulsion.

### Application of the meta-stable o/w emulsions

This sprayable o/w emulsion according to Example 5 turns into a w/o emulsion very quickly during skin application. To characterize the effects of this emulsion after skin application, contact angle measurement, waterproofing efficacy with Sun Protection Factor (SPF) determination, Trans Epidermal Water Loss (TEWL) tests and sensory profiles were performed. The o/w emulsion showed a final conductivity of about 2000 µS/cm (Conductimeter Crison, Model Basic 30, RT) and a viscosity according to Brookfield (RVT, 23 °C, Spindle 5, 10 rpm) of 2,000 mPas.

### 1. Contact Angle Concept

The contact angle of a liquid on a solid depends on the interfacial tensions between the materials involved. The contact angle of water on human skin is a measure of the wettability of the skin. Hydrophilic skin shows high wettability, and the water drop rapidly spreads and forms a low equilibrium contact angle. Hydrophobic skin shows low wettability and the water drop does not spread resulting in a high equilibrium contact angle. The hydrophilicity (wettability) of skin is strongly influenced by application of skin care products. A w/o emulsion applied on the skin shows a high contact angle, which means low wettability, from the beginning of measurements. In contrast, treatment of skin with an o/w emulsion typically leads to fast spreading of water, meaning a low contact angle and high wettability, i.e. low water resistance in sun care.

However, every o/w emulsion applied on skin tends to invert to a w/o emulsion due to water evaporation over time. 200 µl of emulsion to be tested were applied on the finger tip. 3 µl of water/min were dropped on the finger tip and the contact angle was measured (water was absorbed with a tissue after every measure). 8 runs were performed. Finally, the contact angle was plotted as a function of time. The kinetic study showed an inflection point that indicates the time the o/w emulsion turns into a w/o emulsion. The results are shown in Figure 1: 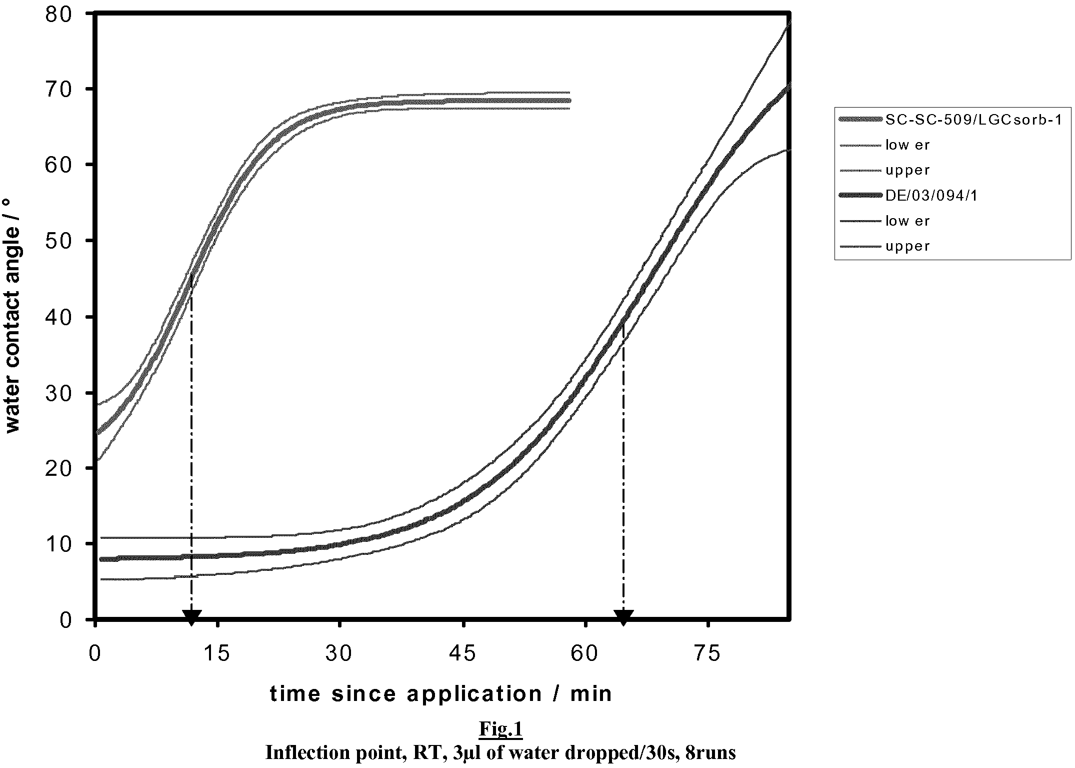 The inflection point for the inventive example is 11.5 minutes; while for the standard o/w emulsion¹ (ref#DE/03/094/1) it is 68.2 minutes. The composition according to the present invention shows significantly faster inversion from o/w to w/o, compared with the standard o/w emulsion.
¹ Ingredients: Lauryl Glucoside (and) Polyglyceryl-2 Dipolyhydroxystearate (and) Glycerin 3,00; Caprylic/Capric Triglyceride 2,00; 2-Ethyl Hexyl Stearate 2,00; Dicaprylyl Carbonate 2,00; Paraffin oil 6,00; Tocopheryl Acetate 0,10; Dimethicone 0,20; Glycerin 3,00; Sodium Polyacrylate 0,70; Deionized water add to 100.

### 2. Trans Epidermal Water Loss (TEWL) test

This study had the primary objective to evidence the effect of several cosmetic formulations on TEWL, on volunteers after one single standardized application. The protocol consisted in applying 3µl/cm² of product (single standardized application) on the forearm of 11 volunteers aged from 18 to 46. TEWL was measured with a Tewameter® at T0, T1h, T2h, T4h and T24h. A TEWL decrease characterizes a decrease of water exchanges from the epidermis to the external environment. This could either mean an improvement of the skin's barrier function or an occlusive effect of the product. However, when the TEWL decrease is still present 24h after application, the hypothesis of the skin's barrier function improvement is more probable. The results are shown in Table 2.

**Table 2**

| **Decrease in TEWL of cosmetic compositions [g*m⁻²*h⁻¹]** | | | | | |
|---|---|---|---|---|---|
| **Time [h]** | **0** | **1** | **2** | **4** | **24** |
| Non-treated | 11.5 | 11.1 | 10.9 | 10.6 | 10.7 |
| Standard w/o emulsion | 11.7 | 11.5 | 10.9 | 9.9 | 9.7 |
| meta-stable o/w emulsion Example 1 | 11.6 | 9.5 | 9.3 | 9.5 | 9.5 |

Compared with a non-treated zone, the inventive meta-stable o/w emulsion induced a significant TEWL decrease one, two, four and twenty-four hours after a single standardized application (from -12% to -16% on average). Under the same application conditions, a standard w/o emulsion did not induce any significant TEWL variations one and two hours after application.

### 3. Determination of sun protection factor and water resistance

The Sun Protection Factor Determination of the tested emulsion according to Example 5 was determined according to the International Sun Protection Factor (SPF) Test Method, COLIPA (12 volunteers aged from 18-56, phototype I, II or III, UV source 300 W Xenon Solar Light). The procedure used for the water resistance test was the shower method with 2 min 30 sec in water at a temperature of 23°C. The water resistance criterion was SPF>50% of the SPF without immersion in the same subjects (10 volunteers). The results are shown in Table 4.

**Table 4**

| **SPF acc. to Colipa, 12 volunteers; Shower test: 23°C tap water 70°, 2min 30s** | | | | | | |
|---|---|---|---|---|---|---|
| **Product** | **SPF** (mean) | **SPF** (labelled) | **SD** | **95 % CI** | **PCD** | **Water Resistance** |
| Emulsion acc. Example 1 | *Before immersion* | | | | | |
| | 26.2 | 25.0 | 6.1 | 21.8-30,5 | high | |
| | *After immersion* | | | | | |
| | 15.3 | | 13.2 | 13.0-17.6 | | yes |

On the basis of the average SPF value of 26.2 determined, the recommended maximum-labelled SPF (Colipa Recommendation N°11) for the test product is a SPF 25. The result for the test product after water immersion evidences that this formulation complies with the water resistance criteria.

### 4. Sensory test

To evaluate the sensory skin perception, the emulsion according to Example 5 and a sprayable sun care benchmark were tested on 11 trained test persons (expert panel of women in the age of 25 to 50) in a sensory assessment. The testing procedure took place in an air-conditioned room at 22°C and with 40 % relative humidity. The results are shown in Figure 2: 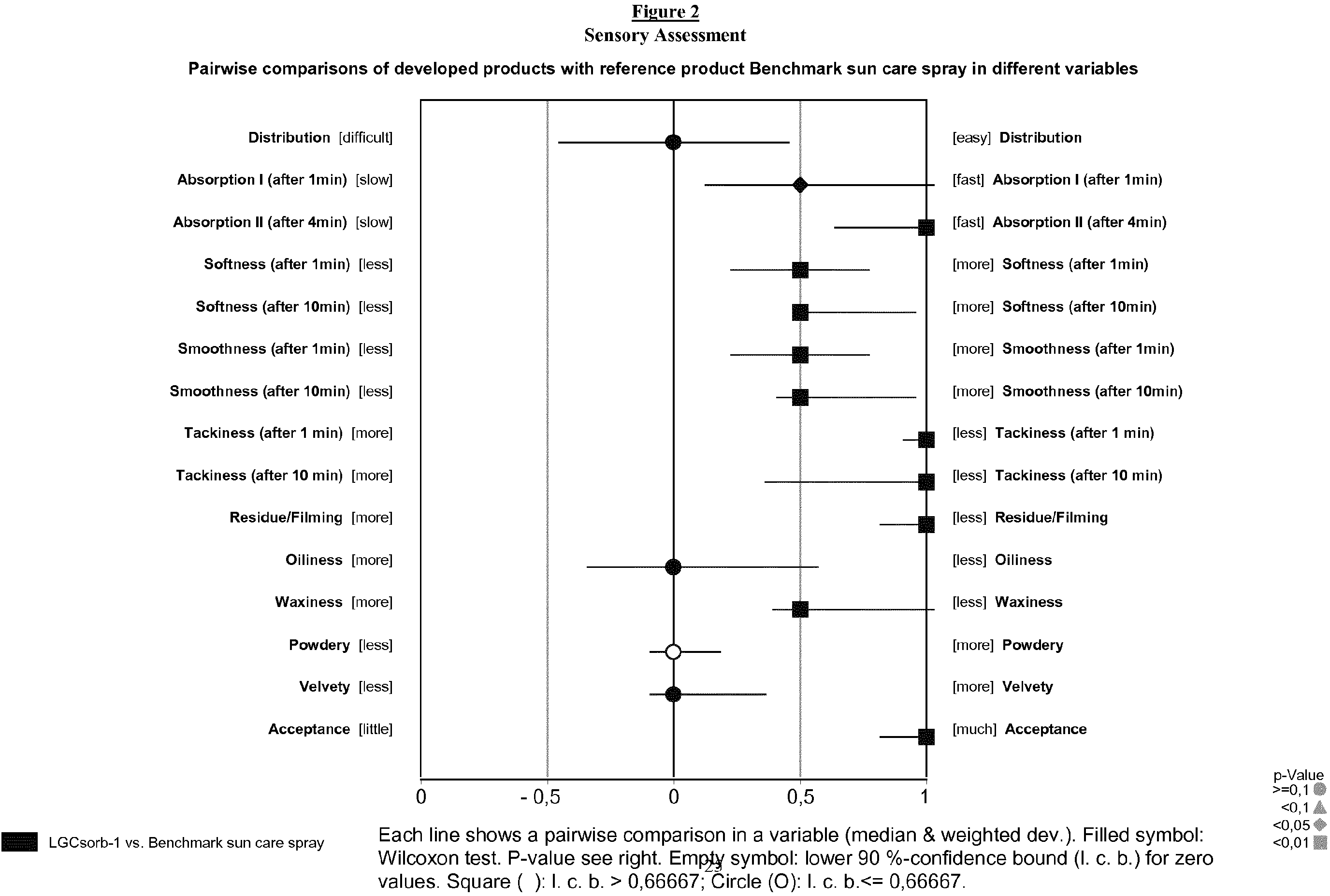 In sun care products, parameters like absorption, tackiness, softness and smoothness are very important. The sensory comparison test of inventive Example 5 with a benchmark sun care spray product of the market shows an improved acceptance for the inventive product with clearly less tackiness, faster absorption, higher softness and smoothness.

## Claims

1. Process for the preparation of aqueous meta-stable o/w emulsions with the ability to invert into w/o emulsions by introduction of mechanical energy, obtainable by the following method:
(i) oil components, w/o emulsifiers and optionally oil-soluble actives are mixed at temperatures in the range from 50 to 90° C to form an oil phase I,
(ii) anionic surfactants, selected from the group consisting of acyl glutamates, alk(en)yl oligoglycoside carboxylates, alk(en)yl polyalkyleneglycolether citrates and their mixtures, are diluted in water at temperatures in the range from 50 to 90° to form an aqueous phase, and this phase is maintained at these temperatures (II)
(iii) phase (I) is added to phase (II) or, alternatively, phase II is added to phase I while maintaining these temperatures in order to obtain an o/w emulsion,
(iv) said o/w emulsion is cooled down to room temperature,
(v) polymeric stabilisers and optionally water soluble actives are added, and
(vi) the final emulsion is homogenised.

2. Process for the preparation of aqueous meta-stable o/w emulsions with the ability to invert into w/o emulsions by introduction of mechanical energy, obtainable by the following method:
(i) oil components, w/o emulsifiers and optionally oil-soluble actives and/or polymeric stabilisers are mixed at temperatures in the range from 50 to 90° to form an oil phase I,
(ii) anionic surfactants selected from the group consisting of acyl glutamates, alk(en)yl oligoglycoside carboxylates, alk(en)yl polyalkyleneglycolether citrates and their mixtures and optionally polymeric stabilisers are diluted in water at temperatures in the range from 50 to 90° to form an aqueous phase (II),
(iii) phase (I) is added to phase (II) while maintaining these temperatures in order to obtain an o/w emulsion,
(iv) said o/w emulsion is cooled down to room temperature,
(v) optionally water soluble actives are added, and
(vi) the final emulsion is homogenised
on condition that said polymeric stabilisers are present in the oil or aqueous phase or both.

3. Processes according to Claim 1 or 2, **characterised in that** the oil components are selected from the group consisting of tri- or partial glycerides, dialkyl ethers, dialkyl carbonates, liposoluble UV filters and their mixtures.

4. Processes according to Claim 1 or 2, **characterised in that** the w/o emulsifiers represent sorbitol esters and/or polyglycerol esters.

5. Processes according to Claim 1 or 2, **characterised in that** the actives are selected from the group consisting of antioxidants, pigments, plant extracts and UV filters.

6. Processes according to Claim 1 or 2, **characterised in that** the polymeric stabilisers are selected from the group consisting of polyacrylates and polysaccharides.

## Patentansprüche

1. Verfahren zur Herstellung von wässrigen metastabilen O/W-Emulsionen mit der Fähigkeit zur Inversion zu W/O-Emulsionen durch Eintragen von mechanischer Energie, die nach der folgenden Methode erhältlich sind:
(i) Ölkomponenten, W/O-Emulgatoren und gegebenenfalls öllösliche Wirkstoffe werden bei Temperaturen im Bereich von 50 bis 90 °C gemischt, was eine Ölphase I ergibt,
(ii) anionische Tenside aus der Gruppe bestehend aus Acylglutamaten, Alk(en)yloligoglycosid-carboxylaten, Alk(en)ylpolyalkylenglykol-ethercitraten und Mischungen davon werden bei Temperaturen im Bereich von 50 bis 90° in Wasser verdünnt, was eine wässrige Phase ergibt, und diese Phase wird bei diesen Temperaturen gehalten (II),
(iii) Phase (I) wird zu Phase (II) gegeben, oder alternativ dazu wird Phase II zu Phase I gegeben, wobei diese Temperaturen aufrechterhalten werden, was eine O/W-Emulsion ergibt,
(iv) die O/W-Emulsion wird auf Raumtemperatur abgekühlt,
(v) polymere Stabilisatoren und gegebenenfalls wasserlösliche Wirkstoffe werden zugegeben, und
(vi) die fertige Emulsion wird homogenisiert.

2. Verfahren zur Herstellung von wässrigen metastabilen O/W-Emulsionen mit der Fähigkeit zur Inversion zu W/O-Emulsionen durch Eintragen von mechanischer Energie, die nach der folgenden Methode erhältlich sind:
(i) Ölkomponenten, W/O-Emulgatoren und gegebenenfalls öllösliche Wirkstoffe und/oder polymere Stabilisatoren werden bei Temperaturen im Bereich von 50 bis 90 °C gemischt, was eine Ölphase I ergibt,
(ii) anionische Tenside aus der Gruppe bestehend aus Acylglutamaten, Alk(en)yloligoglycosid-carboxylaten, Alk(en)ylpolyalkylenglykol-ethercitraten und Mischungen davon und gegebenenfalls polymere Stabilisatoren werden bei Temperaturen im Bereich von 50 bis 90° in Wasser verdünnt, was eine wässrige Phase (II) ergibt,
(iii) Phase (I) wird zu Phase (II) gegeben, wobei diese Temperaturen aufrechterhalten werden, was eine O/W-Emulsion ergibt,
(iv) die O/W-Emulsion wird auf Raumtemperatur abgekühlt,
(v) gegebenenfalls werden wasserlösliche Wirkstoffe zugegeben, und
(vi) die fertige Emulsion wird homogenisiert,
mit der Maßgabe, dass die polymeren Stabilisatoren in der Ölphase oder in der wässrigen Phase oder in beiden Phasen vorliegen.

3. Verfahren nach Anspruch 1 oder 2, **dadurch gekennzeichnet, dass** die Ölkomponenten aus der Gruppe bestehend aus Tri- oder Partialglyceriden, Dialkylethern, Dialkylcarbonaten, fettlöslichen UV-Filtern und Mischungen davon ausgewählt werden.

4. Verfahren nach Anspruch 1 oder 2, **dadurch gekennzeichnet, dass** es sich bei den W/O-Emulgatoren um Sorbitolester und/oder Polyglycerolester handelt.

5. Verfahren nach Anspruch 1 oder 2, **dadurch gekennzeichnet, dass** die Wirkstoffe aus der Gruppe bestehend aus Antioxidantien, Pigmenten, Pflanzenextrakten und UV-Filtern ausgewählt werden.

6. Verfahren nach Anspruch 1 oder 2, **dadurch gekennzeichnet, dass** die polymeren Stabilisatoren aus der Gruppe der Polyacrylate und Polysaccharide ausgewählt werden.

## Revendications

1. Procédé de préparation d'émulsions aqueuses huile dans eau métastables capables de subir une inversion pour devenir des émulsions eau dans huile par introduction d'énergie mécanique, pouvant être obtenues par la méthode suivante :
(i) mélange de constituants de type huile, d'émulsifiants eau dans huile et optionnellement de constituants actifs solubles dans l'huile à des températures de 50 à 90 °C pour former une phase huileuse (I),
(ii) dilution dans de l'eau de tensioactifs anioniques, sélectionnés dans le groupe constitué d'acylglutamates, de carboxylates d'alc(én)yloligoglycoside, de citrates d'alc(én)yléther de polyalkylène glycol et de mélanges de ceux-ci, à des températures de 50 à 90 °C pour former une phase aqueuse, et cette phase étant maintenue à ces températures (II),
(iii) addition de la phase (I) à la phase (II) ou, autrement, addition de la phase (II) à la phase (I) tout en maintenant ces températures afin d'obtenir une émulsion huile dans eau,
(iv) refroidissement de ladite émulsion huile dans eau jusqu'à la température ambiante,
(v) addition de stabilisants polymères et optionnellement de constituants actifs hydrosolubles, et
(vi) homogénéisation de l'émulsion finale.

2. Procédé de préparation d'émulsions aqueuses huile dans eau métastables capables de subir une inversion pour devenir des émulsions eau dans huile par introduction d'énergie mécanique, pouvant être obtenues par la méthode suivants :
(i) mélange de constituants de type huile, d'émulsifiants eau dans huile et optionnellement de constituants actifs solubles dans l'huile et/ou de stabilisants polymères à des températures de 50 à 90 °C pour former une phase huileuse (I),
(ii) dilution dans de l'eau de tensioactifs anioniques sélectionnés dans le groupe constitué d'acylglutamates, de carboxylates d'alc(én)yloligoglycoside, de citrates d'alc(én)yléther de polyalkylène glycol et de mélanges de ceux-ci, et optionnellement de stabilisants polymères à des températures de 50 à 90 °C pour former une phase aqueuse (II),
(iii) addition de la phase (I) à la phase (II) tout en maintenant ces températures afin d'obtenir une émulsion huile dans eau,
(iv) refroidissement de ladite émulsion huile dans eau jusqu'à la température ambiante,
(v) optionnellement, addition de constituants actifs hydrosolubles, et
(vi) homogénéisation de l'émulsion finale,
à condition que lesdits stabilisants polymères soient présents dans la phase huileuse ou dans la phase aqueuse ou dans les deux.

3. Procédés selon la revendication 1 ou 2, **caractérisés en ce que** les constituants de type huile sont sélectionnés dans le groupe constitué de triglycérides ou de glycérides partiels, d'éthers dialkyliques, de carbonates de dialkyle, de filtres UV liposolubles et de mélanges de ceux-ci.

4. Procédés selon la revendication 1 ou 2, **caractérisés en ce que** les émulsifiants eau dans huile représentent des esters de sorbitol et/ou des esters de polyglycérol.

5. Procédés selon la revendication 1 ou 2, **caractérisés en ce que** les constituants actifs sont sélectionnés dans le groupe constitué d'antioxydants, de pigments, d'extraits végétaux et de filtres UV.

6. Procédés selon la revendication 1 ou 2, **caractérisés en ce que** les stabilisants polymères sont sélectionnés dans le groupe constitué de composés polyacrylates et de polysaccharides.
